# EUROPÄISCHE PATENTSCHRIFT

(11) **EP 1 274 378 B1**
(45) Veröffentlichungstag und Bekanntmachung des Hinweises auf die Patenterteilung: **28.12.2005**
(21) Anmeldenummer: 00914045.0
(22) Anmeldetag: 25.02.2000
(51) Int. Cl.: A61F 13/12

(54) **PFLASTER ZUM AUFBRINGEN AUF DER HAUT MIT VORSPRÜNGEN AUF DEM PFLASTER**
PLASTER WITH PROTRUSIONS FOR APPLICATION TO THE SKIN
PANSEMENT ADHESIF DOTE DE PROTUBERANCES ET DESTINE A ETRE APPLIQUE SUR LA PEAU

(30) Priorität: 02.03.1999 DE 29903694 U; 30.03.1999 DE 29905808 U; 21.05.1999 DE 19924842
(43) Veröffentlichungstag der Anmeldung: 15.01.2003
(73) Patentinhaber: Meyer-Schwarz, Tatjana, 65589 Hadamar (DE); Schwarz, Volker, 65589 Hadamar (DE); Strass, Michaela, 56412 Heilberscheid (DE)
(72) Erfinder: Meyer-Schwarz, Tatjana, 65589 Hadamar (DE); Schwarz, Volker, 65589 Hadamar (DE); Strass, Michaela, 56412 Heilberscheid (DE)
(74) Vertreter: Walter, Wolf-Jürgen
(86) Internationale Anmeldenummer: PCT/DE2000/000568
(87) Internationale Veröffentlichungsnummer: WO 2000/051536

(56) Entgegenhaltungen:
- EP-A- 0 875 223
- WO-A-97/46265
- DE-A- 4 431 918
- FR-A- 2 679 129

## Beschreibung

Die Erfindung betrifft ein Pflaster zum Aufbringen auf Haut mit Vorsprüngen auf dem Pflaster, wodurch es als Anti-Faltenpflaster wirken kann.

Aus der.DE 3207797 A1 ist ein Creme-Halbmond bekannt, um Falten, unterhalb der Augen zu behandeln. Die Creme-Halbmonde können gemäß dieser Druckschrift als Pflaster ausgebildet sein und weisen geeignete Pflegesubstanzen auf, um Falten zu vermindern oder rückzubilden.

Weiterhin sind gemäß EP-A-214368 Pflaster bekannt zur Steigerung und Stabilisierung der Hauttemperatur. Diese Pflaster bestehen aus einer wärmeisolierenden Schaumstoffschicht, einer wärmeleitenden Metallschicht, einer weiteren Schaumstoffschicht sowie einer oder mehreren in diese Schaumstoffschicht eingelagerten, streifenförmig abwechselnd positiv und negativ dauerhaft magnetisierten Kunststoffolien. Durch die durch Magnetfelder erzeugte Wärme erhält man eine Stabilisierung der erhöhten Hauttemperatur.

Weiterhin ist aus der DE-A-4240952 ein Kompressionsverband bekannt, bei dem die Innenseite des Verbandes Noppen trägt, die bestimmten Akupressurpunkten zugeordnet sind.

Die WO97/46265 offenbart ein medizinisches Wundpflaster mit einer Schicht, die ein bioresorbierbares Material enthält und einer Deckschicht aus wasserdampfdurchlässigem Material.

Aus der FR-A-2679129 ist ein Pflaster zum Schutz von frischen Operationsnarben/wunden bekannt, bestehend aus zwei Polstern aus stoßdämpfendem Material, die die dazwischenliegende Narbe schützen soll. Die EP-A-875223 beschreibt eine medizinische Muskelscheidenbandage in Form eines Bandes, das auf der Innenseite Taschen zur Aufnahme von Spindeln hat, um bei Körperbewegungen Vibrationen zwecks Aktivierung von Nervenkreisläufen auf das Gewebe und die Haut auszuüben. Die DE 4431918 beschreibt ein Pflaster mit Noppen an einem Luftpolster, wobei die Noppen eine punktförmige Auflage auf der Haut bilden und die Hautdurchblutung fördern.

Der Erfindung liegt die Aufgabe zugrunde, ein Pflaster bereitzustellen, das durch eine besondere Gestaltung zur Rückbildung von Hautfalten insbesondere im Gesicht beiträgt und einfach zu handhaben ist sowie die Verwendung des Pflasters in einem kosmetischen Verfahren.

Erfindungsgemäß ist das kosmetische Pflaster zum Aufbringen auf menschliche Haut mit einer Haftschicht oder sekundären Befestigungen und mit Vorsprüngen auf der der Haut zugewandten Innenseite des Pflasters dadurch gekennzeichnet, daß der oder die Vorsprünge durch einen länglichen Vorsprung oder zwei bis vier dicht beieinander und im wesentlichen parallel zueinander angeordnete längliche Vorsprünge verkörpert werden, die als elastisches, komprimierbares Druckpolster für die Haut und das darunterliegende Bindegewebe ausgebildet sind, und das Pflaster aus einem thermisch isolierenden Material besteht oder eine thermisch isolierende Schicht zwischen der Haftschicht und den anderen Teilen des Pflasters angeordnet ist. Die länglichen Vorsprünge entsprechen in ihrer Länge etwa der Länge der Hautfalten.

Unter "Pflaster" wird allgemein ein Trägerelement verstanden, auf der die genannten länglichen Vorsprünge angeordnet bzw. ausgebildet sind.

Als Befestigungsmittel wird vorzugsweise eine Selbstklebeschicht verwendet, die auf dem Trägermaterial für das Pflaster oder auf dem thermisch isolierenden Material angeordnet ist. Die Haftschicht kann teilweise unterbrochen sein, z.B. kann sie wenigstens beiderseits der parallelen Vorsprünge und parallel zu diesen angeordnet sein. Befestigungen des Pflasters mit Bändern sind ebenfalls möglich.

Eine Ausführungsform der Erfindung besteht darin, daß die thermisch isolierende Schicht zwischen der Unterseite der Vorsprünge und der Selbstklebeschicht einerseits und einer Trägerschicht andererseits angeordnet ist. Die Erhebungen bleiben damit frei von Klebemitteln.

Eine weitere Ausführungsform der Erfindung besteht darin, daß die thermisch isolierende Schicht zwischen der Selbstklebeschicht einerseits und der Oberseite der Vorsprünge und einer Trägerschicht andererseits angeordnet ist. In diesem Falle würde die Selbstklebeschicht auch die Vorsprünge abdecken.

Die thermisch isolierende Schicht kann perforiert sein. Sie kann auch durch ein Aufdampfverfahren von z.B. Silber auf eine Trägerschicht entstanden sein. Andere konventionelle thermisch isolierende Materialien mit der notwendigen Flexibilität können eingesetzt werden. Ein "Pflaster aus einem thermisch isolierenden Material" erfaßt auch ein beliebiges Trägermaterial mit darüber angeordneter thermisch isolierender Schicht. Thermisch isolierend ist ein solches Material, das bei einem Temperaturgradienten zwischen Haut- und Umgebungstemperatur von 10-20 °C einen Temperaturabfall von weniger als 5 °C gewährleistet.

Unter "länglicher Vorsprung" im Zusammenhang mit der vorliegenden Erfindung ist ein aus der Ebene heraustretender Rücken mit vorgegebener Höhe zu verstehen, dessen Länge ein Mehrfaches von seiner Breite beträgt.

Die Vorsprünge können im Querschnitt vorzugsweise quadratisch, rechteckig, kreissegmentförmig, kreisförmig, tropfenförmig oder trapezförmig ausgebildet sein.

Das Pflaster ist vorteilhaft derart aufgebaut, daß eine erste Schicht als wärmeisolierende schicht ausgebildet ist. Diese oder wenigstens eine weitere Schicht kann als wenigstens teilweise selbstklebende Schicht ausgebildet sein. Eine dieser Schichten oder eine weitere Schicht ist derart ausgebildet, daß sie wenigstens einen sich in Längsrichtung erstreckenden Vorsprung aufweist.

Es ist auch möglich, diesen Vorsprung, der als längliches Druckpolster ausgebildet sein kann, als einzelne Schicht oder einzelnes Druckpolster auszubilden, derart, daß die Schicht oder das Druckpolster erst auf der Haut aufgebracht wird und anschließend das Pflaster mit der wärmeisolierenden Schicht darüberliegend angeordnet ist. Beispielsweise können gemäß der Erfindung zwei länglich ausgebildete Vorsprünge oder Druckpolster vorgesehen sein, die rechts und links neben der zu behandelnden Hautfalte und im wesentlichen parallel dazu angeordnet sind. Im allgemeinen haben diese Vorsprünge, sofern es sich um zwei oder mehrere nebeneinander handelt, jeweils einen Abstand voneinander im Bereich von 0,1 bis über 50 mm, vorzugsweise 1 bis 40 mm.

Unter "im wesentlichen parallel dazu" wird verstanden, daß die beiden Vorsprünge näherungsweise dem Verlauf der Falte, die auch eine Narbe sein kann, auf beiden Seiten der Falte in etwa dem gleichen Abstand zur Falte folgen.

Unter "Hautfalten" im Sinne der Erfindung werden dauerhafte, schmale, wellenartige Hautvertiefungen oder vertiefte Narbenbildungen verstanden, deren Schenkel im wesentlichen parallel oder sternförmig zueinander verlaufen und die im wesentlichen geradlinig oder leicht gebogen in der Haut verlaufen. Längliche Vorsprünge mit der Länge von Hautfalten sind insbesondere solche, deren Länge im Bereich von 0,5 bis 12 cm liegt.

Auf einem Pflaster können mehrere einzelne längliche Vorsprünge und/oder mehrere parallel zueinander stehende längliche Vorsprünge entsprechend dem Faltenbild einer menschlichen Gesichtshaut angeordnet sein. Die Vorsprünge können auch am Rand eines Pflasters angeordnet sein, und die restliche der Haut zugewandte Fläche kann als Haftschicht ausgebildet sein.

Eine weitere Ausführungsform der Erfindung besteht darin, daß die länglichen Vorsprünge zumindest teilweise aus einem Poly(natriumpolyacrylat)-Homopolymeren bestehen, das bei Kontakt mit Feuchtigkeit sein Volumen um ein Mehrfaches vergrößert, z. B auf das 3 bis 8-fache. Ein solches Polymeres ist beispielsweise als AQUA KEEP® von sumitomo seika Chemicals Co. Ltd, Osaka, Japan erhältlich. Durch die Volumenvergrößerung kann der Druck auf die darunter liegende Haut vergrößert oder im Zusammenhang mit der Elastizität eines elastischen Materials gezielt eingestellt werden.

Es kann auch ein beliebiges anderes Material für die Vorsprünge gewählt werden, das sich bei Wärmeeinfluß ausdehnt oder aus einem entspannten Zustand in einen Spannungszustand übergeht und dabei im wesentlichen senkrecht Druck auf die Haut neben der Falte ausübt.

Die länglichen Vorsprünge können in ihrer Höhe durchgehend konstant sein; sie können auch durch Querrillen oder Absenkungen in Querrichtung unterbrochen sein.

Das Pflaster wird derart auf die Haut geklebt oder in anderer Weise befestigt, daß die länglichen Vorsprünge etwa parallel zur Hautfalte und neben der Hautfalte liegen und einen bestimmten geringen oder starken Druck auf die Haut bzw. das darunterliegende Bindegewebe senkrecht oder senkrecht und seitlich ausüben. Durch den Druck, der im Bereich der Problemzone (Falte) ausgeübt wird, werden Gewebe und Gewebsflüssigkeit in Richtung der Falten verschoben, so daß sich die Falten zurückbilden können. Durch Wärmeeinwirkung wird Lymphflüssigkeit transferabel gemacht und durch den Druck das Austreten der Lymphflüssigkeit aus Lymphbahnen (Gewebe, Zellen) in benachbarte Lymphbahnen(Gewebe, zellen) mit reduziertem Flüssigkeitsgehalt veranlaßt. Eine Beseitigung oder Reduzierung der Falten kann erreicht werden, wenn das Gewebe unter der Falte mit Lymphflüssigkeit weitgehend aufgefüllt wird. Es handelt sich dabei insbesondere um Falten, die sich z.B. durch ständige mimische Bewegung gebildet haben und bei denen die Zellen aus dem Faltenbereich retardiert oder in den daneben liegenden Bereich des Bindegewebes gedrängt worden sind. Es kann sich auch um Muskulaturveränderungen handeln sowie um Narben oder Narbenränder.

Darüber hinaus wird durch die körpereigene Wärme, die infolge Anordnung der thermisch isolierenden Schicht (Thermofolie) des erfindungsgemäßen Pflasters nicht nach außen entweichen kann, sondern im Bereich des Pflasters erhalten bleibt, die Muskulatur vollkommen entkrampft und entspannt. Durch diese Wärmebehandlung werden zusätzlich Poren der Haut geöffnet. Selbstverständlich kann auch Wärme von außen zugeführt werden, z.B. durch Bestrahlung, übliche Wärmeträger, Vibration (Massage-Effekt durch Handmassage oder Vibrationsgerät regelmäßig oder pulsierend) oder durch eine in dem Vorsprung herbeigeführte exotherme chemische Reaktion. Auch ein vorheriger kurzer lokaler Kälteschock kann eine anschließende Erwärmung der entsprechenden Hautpartien begünstigen.

In einer bevorzugten Ausführungsform der Erfindung weist das Pflaster vorteilhaft noch eine kosmetische Zusammensetzung auf, insbesondere im Faltenbereich, wobei Antifalten-, Vitamin- und/oder östrogenmittel, Retinolderivate, Nanosome, Episome und ähnliche Präparate bevorzugt sind, die durch die geöffneten Poren der Haut leichter und einfacher sowie gezielt und effektiv von der Haut direkt an der Problemzone aufgenommen werden können. Es können auch farbkorrigierende Substanzen, wie Selbstbräunungsmittel z.B. auf Basis von Dihydroxyaceton oder dessen Vorläufern zusätzlich in einer kosmetischen Zusammensetzung enthalten sein.

Das Pflaster hat den Vorteil, daß es einfach in der Handhabung ist und auf beliebige Problemzonen aufgeklebt werden kann.

Das erfindungsgemäße Pflaster bewirkt durch den Druck zusätzlich eine Stimulation von Stoffwechselorganen sowie durch die Wärme eine Entkrampfung der Muskulatur sowie eine Porenöffnung.

Die Vorsprünge des erfindungsgemäßen Pflasters bestehen aus einem elastischen Material, d. h. aus einem Material, das nach Zusammendrücken bestrebt ist, wieder in die Ausgangsposition zurückzukehren oder in sonstiger Weise durch Ausdehnung Druck auf das darunter liegende Gewebe ausüben kann. Da die Trägerfläche des Pflasters nach dem Zusammendrücken der Vorsprünge auf der Haut befestigt wird, kann das Material nicht mehr anderweitig ausweichen, wodurch ein Druck auf die entsprechende Hautpartie durch die komprimierten Vorsprünge ausgeübt wird.

Die Vorsprünge können beispielsweise aus elastischen oder weichelastischen Schaumstoffstreifen oder aus plastischen oder festen Streifen bestehen. Diese Streifen können auf der Klebefläche des Pflasters aufgebracht sein. Es ist auch möglich, ein anderes Material, beispielsweise Neopren° zu verwenden. Wichtig ist, daß das Material weich, hautfreundlich und z.B. feinporig ist.

Die Vorsprünge können auch als Depot für einen Wirkstoff ausgebildet sein, oder sie können lufteinlaßbare und luftauslaßbare elastische Hohlkörper sein.

Die Vorsprünge können auch aus einem schweißaufsaugenden Material bestehen oder es enthalten.

Eine bevorzugte Ausführungsform besteht darin, daß ein elastischer Schaumstoff gewählt wird, der einen Gleichgewichtsdruck bei 40 % Kompression im Bereich von 50 bis 400 p/cm² hat. Gleichgewichtsdruck bei 40 % Kompression bedeutet, daß ein Schaumstoff bis zu etwa 60 % seiner Höhe komprimiert wird, dann in eine Instron-Universalprüfgerät zwischen die parallelen Flächen eines Probenhalters gebracht wird, wobei die Flächen einen Abstand voneinander haben, der 40 % der Probenhöhe entspricht. Das Gerät ist weiterhin mit einer Druckmeßdose ausgestattet. 10 Minuten nach dem Einbringen der komprimierten Probe in den Probenhalter und Anlegen der Probe an beide Platten des Probenhalters, wird der von dem oder den Probekörper(n) ausgeübte Druck gemessen und ins Verhältnis zur Probefläche gesetzt, wodurch sich der Gleichgewichtsdruck ergibt.

Ein Gleichgewichtsdruck von 50 bis 500 p/cm² ist für die erfindungsgemäße Wirkung besonders vorteilhaft.

Die erfindungsgemäßen Vorsprünge in Form von Druckpolstern können durch eine oder mehrere längs darin angeordnete und im wesentlichen nicht elastische Einlagen verstärkt sein. Dies kann beispielsweise in Form eines metallischen Drahtes oder eines Kunststoffdrahtes erfolgen. Eine solche Verstärkung ist vorzugsweise verformbar ausgebildet, um diese der Oberflächenform der Hautpartie anzupassen und einen gleichmäßigen Druck oder, wenn gewollt an einer oder mehreren Stellen einen größeren Druck auszuüben. Wahlweise kann der Vorsprung auch durch ein Metallstück, insbesondere ein länglich geformtes Metallstück versehen sein, um den Druck auf die Haut an der jeweiligen Stelle noch weiter zu verstärken. Das Metallstück kann magnetisch oder superparamagnetisch sein.

Der Draht kann vorzugsweise verformbar sein.

Die Klebefläche des erfindungsgemäßen Pflasters kann wie bei den zum Stand der Technik gehörenden Pflastern ausgebildet sein, d.h. mit den üblichen Heißschmelzklebemassen ausgestattet sein.

Das Pflaster weist vorzugsweise Felder auf, die eine kosmetische Zusammensetzung insbesondere eine Anti-Faltenmittel aufweisen, oder auf denen ein solches Mittel aufgetragen wer- den kann. Bevorzugt sind diese Felder an den Stellen vorgesehen, mit denen der direkte Kontakt zur Hautfalte gegeben ist. Hierdurch kann die Wirkung des Pflasters erhöht werden. Eingesetzt werden können kosmetische Zusammensetzungen der Gruppe, die aus kosmetischen Cremes, Gelen, Salben, Lotionen, Pudern, Wässern, ölen und Gemischen davon besteht. Es können auch pharmazeutisch wirksame Substanzen aufgetragen werden.

Das erfindungsgemäße Pflaster hat den Vorteil, daß es einfach in der Handhabung ist, und das es einen einfachen Aufbau aufweist, wodurch es auch in der Herstellung preisgünstig ist.

Das erfindungsgemäße Pflaster kann speziell ausgebildet sein als Nasenpflaster, Oberlippenpflaster, Stirnpflaster oder dergleichen, je nachdem, welcher Faltenbereich behandelt werden soll. Das Pflaster kann auch entsprechend den jeweiligen Gesichtsformen vorgeformt sein, Schlitze oder Einschnitte enthalten oder in anderer Weise der Körperform anpaßbar gestaltet sein.

Der wenigstens eine längliche Vorsprung des Pflasters kann ebenfalls unterschiedlich ausgebildet sein. Es ist zum einen denkbar, nur einen Vorsprung vorzusehen, beispielsweise zur Anwendung im Augenbereich oder im Oberlippenbereich. Der längliche Vorsprung kann gerade sein oder gekrümmt, um bestimmten Faltenverläufen zu folgen.

Für Falten im Wangen oder Stirnbereich können, wie schon ausgeführt, z.B. zwei streifen aus z.B. elastischem Material auf der Trägerfolie als Vorsprünge vorgesehen sein. Diese können getrennt voneinander angeordnet sein oder als ein Streifen ausgebildet sein mit einer Vertiefung in der Mitte, so daß sich ein wellenförmiger Querschnitt ergibt. Der Querschnitt des Druckpolsters allgemein kann kreissegmentförmig, rund, eckig z. B. dreieckig oder trapezförmig oder auch in anderen Formen vorgesehen sein.

Eine weitere Ausführungsform besteht darin, daß der Vorsprung oder die Vorsprünge leicht lösbar mit dem Pflaster verbunden sind. Dadurch erhält man ein sogenanntes Baukastensystem, d.h. die Vorsprünge können einzeln an beliebiger Stelle auf der Haut neben der Falte aufgelegt oder aufgeklebt werden, und anschließend wird das Trägermaterial des Pflasters darübergelegt und befestigt. Auch hier kann das Trägermaterial das thermisch isolierende Material selbst oder ein Gewebe sein, das zusätzlich mit einer thermisch isolierenden Schicht versehen ist. Sowohl die festen als auch die leicht lösbaren Vorsprünge können beliebige vorgeformte Querschnitte und Längen haben und auch elastische Hohlkörper mit kleiner öffnung darstellen, die nach dem Zusammendrücken des Hohlraumes und Befestigen des Trägermaterials darüber das im Inneren gebildete Vakuum durch wieder eingezogene Luft auffüllen und dadurch Druck auf die darunter liegende Haut ausüben.

Eine weitere Ausführungsform der Erfindung besteht darin, als Trägermaterial für das Pflaster, das zugleich das thermisch isolierende Material sein kann, ein zumindest teilweise transparentes Material einzusetzen. Dadurch wird die Anordnung der länglichen Vorsprünge neben den Hautfalten durch eine optische Kontrolle erleichtert.

Wenn gleichzeitig die Vorsprünge mit einem zusätzlichen Material versehen sind, das bei einer Temperatur im Bereich von 30-40 °C seine Farbe ändert, kann der Anwender durch den Farbwechsel, der nach Aufbringen des so vorbereiteten Pflasters auf die Haut auftritt, einen optischen Hinweis für die einsetzende Wirkung erhalten.

Der längliche Vorsprung auf dem Pflaster ist wenigstens 15 mm lang, vorzugsweise 20 bis 140 mm.

Der längliche Vorsprung kann 0,3 bis 50 mm breit sein, vorzugsweise 0,5 bis 15 mm.

Der längliche Vorsprung ist unkomprimiert etwa 0,2 bis 50 mm hoch und läßt sich z.B. bis auf 2 % seiner ursprünglichen Höhe komprimieren. Durch gezielte Wahl des elastischen Materials durch den Fachmann kann der ausgeübte Druck auf die entsprechende Hautfläche vom Grundsatz her beeinflußt werden (Maximum) und vom Anwender gewählt werden (Minimum).

Das erfindungsgemäße Pflaster kann hinsichtlich Trägermaterial, Thermofolie und Selbstklebeschicht vorzugsweise aus einem oder mehreren wasserdampfdurchlässigen Materialien bestehen.

Das erfindungsgemäße Pflaster hat darüber hinaus die zusätzliche positive Sekundärwirkung, daß es entkrampfende und entspannende Eigenschaften aufweist. Dadurch, daß eine Druck- und Wärmewirkung des Pflasters auf die betreffenden Hautpartien ausgeübt wird, treten die entkrampfenden und entspannenden Eigenschaften auf. Diese Eigenschaften unterstützen die Wirkung des Pflasters. Durch die Druckwirkung tritt zusätzlich ein Effekt auf, daß das im Gesicht angeordnete Pflaster mimisch bedingte schlechte Angewohnheiten bewußt macht und damit dem Träger zur bewußten Änderung seiner Gewohnheiten verhelfen kann.

Die gegebenenfalls auf dem Pflaster angeordneten Kosmetikprodukte, wie Anti-Faltencremes, sind vorzugsweise High-Tech-Antifaltenprodukte bekannter Kosmetikhersteller, welche Retinol in Superliposomen verpackt aufweisen oder andere geeignete Transportsysteme. Diese Produkte sind stabil, wirksam und im wesentlichen ohne Nebenwirkungen. Weiterhin können Kosmetikprodukte verwendet werden, die den Angriff freier Radikale abwehren, die Hautschäden verhindern und/oder reparieren, zum Beispiel solche mit Radikalfängern, wie Vitamin C und Vitamin E sowie deren Derivaten, Superoxiddismutase, Gallensäurederivate, Karotine, EDTA und deren Derivate. Auch Produkte mit Lipiden und Chitosanen können eingesetzt werden. Die Wirkung kann weiter unterstützt werden durch Feuchthaltemittel in kosmetischen Zusammensetzungen, die die Porenöffnung durch Wärme und Feuchtigkeit unterstützen

Ein weiterer Gegenstand der Erfindung ist die Verwendung eines Pflasters der oben beschriebenen Art zum Aufkleben auf die Haut in der Weise, daß der wenigstens eine längliche Vorsprung in Nähe einer Hautfalte, insbesondere einer markanten Hautfalte und im wesentlichen parallel zu dieser Hautfalte angeordnet wird und das Pflaster mit der Innenseite in dieser Stellung angedrückt und für einen Zeitraum von 30 Minuten bis 24 Stunden befestigt, insbesondere festgeklebt wird.

Ein weiterer Gegenstand der Erfindung ist die Verwendung des erfindungsgemäßen Pflasters durch Befestigen, insbesondere Aufkleben auf die Haut in der Weise, daß zwei im wesentlichen parallel zueinander stehende längliche Vorsprünge links und rechts von einer Hautfalte und im wesentlichen parallel zu dieser Hautfalte angeordnet werden und das Pflaster mit der Innenseite in dieser Stellung angedrückt und für einen Zeitraum von 30 Minuten bis 24 Stunden befestigt wird.

In beiden Fällen beträgt der Zeitraum vorzugsweise 0,5 bis 10 Stunden.

Vorzugsweise erfolgt das Andrücken des Pflasters in beiden Fällen unter gleichzeitiger Kompression des elastischen oder weichelastischen Materials der Vorsprünge.

Im allgemeinen ist das Trägermaterial des Pflasters bzw. das thermisch isolierende Material eine nichtelastische biegsame Folie oder ein entsprechendes Gewebe.

Eine weiterer Gegenstand der Erfindung ist ein kosmetisches Verfahren zur Rückbildung von Hautfalten, dadurch gekennzeichnet, daß man ein erfindungsgemäßes Pflaster in Nähe der Hautfalte und mit den länglichen Vorsprüngen des Pflasters im wesentlichen parallel zu der Hautfalte aufbringt oder durch die Hände oder ein Massagegerät auf die Stellen neben der Hautfalte Druck ausübt oder beide Maßnahmen nacheinander und mehrfach ausführt.

Auf der Zeichnung sind Ausführungsbeispiele der Erfindung dargestellt, und zwar zeigen:
Fig. 1 eine Ausführungsform der Erfindung im Schnitt;
Fig. 2 eine Ausführungsform im Schnitt mit zusätzlicher Kosmetikproduktfläche;
Fig. 3 eine Ausführungsform der Erfindung im Schnitt;
Fig. 4 eine gegenüber Fig. 1 geänderte Ausführungsform im Schnitt;
Fig. 5 eine gegenüber Fig. 3 geänderte Ausführungsform im Schnitt;
Fig. 6 die Anordnung von zwei Pflastern im Bereich des Nasenflügels bis zum Mundwinkel;
Fig. 7 die Anordnung eines Pflasters im Oberlippenbereich und die Anordnung eines Pflasters unterhalb der Augen;
Fig. 8 ein geändertes Ausführungsbeispiel;
Fig. 9 ein geändertes Ausführungsbeispiel;
Fig. 10 eine Ausführungsform mit abgelösten Druckpolstern;
Fig. 11 eine Ausführungsform mit abgelösten Druckpolstern und darüber angeordnetem Trägermaterial;
Fig. 12 eine Ausführungsform mit abgestuften Vorsprüngen zur partiell unterschiedlichen Druckausübung;
Fig. 13 ein Ausführungsform in Vorderansicht mit Absenkungen in Querrichtung bei einem länglichen Vorsprung;
Fig. 14 ein Ausführungsform in Seitenansicht mit Absenkungen in Querrichtung bei einem länglichen Vorsprung;
Fig. 15 eine Ausführungsform mit tropfenförmigen Vorsprüngen vor dem Aufkleben der Haftflächen auf die Haut;
Fig. 16 das aufgeklebte Pflaster gemäß Fig. 15.

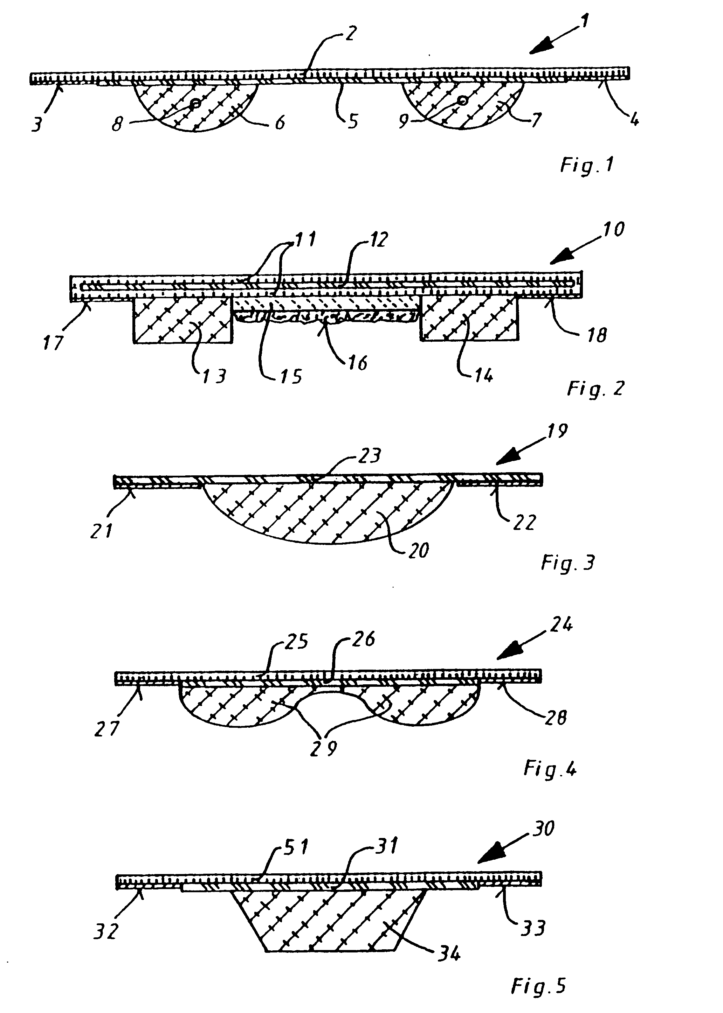

Fig. 1 zeigt ein Pflaster 1 mit einer Textilschicht 2, die in Seitenbereichen Klebeschichten 3, 4 aufweist. Das Pflaster 1 weist darüberhinaus eine Schicht aus einer wärmeisolierenden Metallfolie 5 auf sowie 2 getrennt voneinander vorliegende Vorsprünge 6, 7, in denen für eine weitere spezielle Ausführungsform Drahtverstärkungen 8, 9 angeordnet sind. Gemäß Fig. 2 ist ein Pflaster 10 gezeigt mit einer Textilschicht 11, die eine Thermofolie 12 umschließt. Die Textilschicht 11 trägt zwei Vorsprünge 13, 14, zwischen denen ein Feld 15 angeordnet ist, auf dem eine Anti-Faltencreme 16 aufgebracht ist. Das Pflaster 10 weist im Randbereich Klebeflächen 17, 18 auf.

Fig. 3 zeigt ein Pflaster 19 mit nur einem kreissegmentförmigen länglichen Vorsprung 20 und Klebeflächen 21, 22. Das Pflaster 19 besteht aus einer Trägerschicht, die zugleich eine Thermofolie 23 ist.

Fig. 4 zeigt ein Pflaster 24 mit einer Textilschicht 25, einer Thermofolie 26 und Klebeflächen 27, 28. Das Pflaster 24 weist darüber hinaus einen aus einem Stück bestehenden Vorsprung 29 auf, der in der Mitte vertieft ausgebildet ist.

Gemäß Fig.5 ist ein Pflaster 30 gezeigt, mit einer Textilschicht 51, einer Thermofolie 31 (aufgedampfte Silberschicht), Klebeflächen 32, 33 und einem trapezförmigen Vorsprung 34.

Die Vorsprünge 6, 7; 13, 14; 20, 29, 34 sind aus einem elastischen Material gebildet, beispielsweise Schaumstoff oder Neopren®. Die Vorsprünge haben nach einem Zusammendrücken das Bestreben, wieder in die Ausgangsposition zurückzukehren.

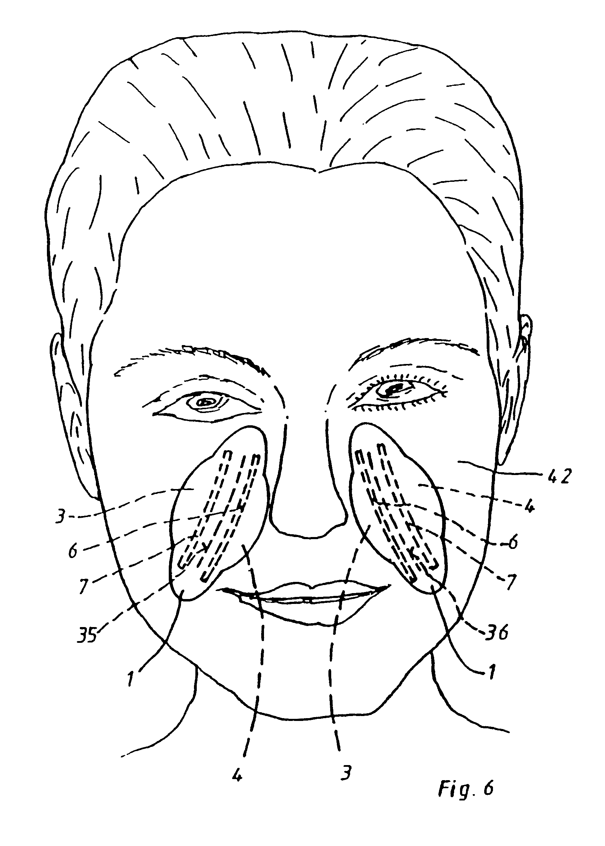

Gemäß Fig. 6 ist die Anordnung des Pflasters 1 in einem Gesicht 42 dargestellt. Die Vorsprünge 6, 7 sind derart angeordnet, daß sie jeweils beidseitig von Falten 35, 36 zu liegen kommen. Durch das Aufkleben der Pflaster 1 werden die Vorsprünge 6, 7 zusammengedrückt. Dadurch, daß die Vorsprünge 6, 7 elastisch ausgebildet sind, haben die als Schaumstoffstreifen ausgebildeten Vorsprünge 6, 7 das Bestreben, wieder in ihre Ausgangslage zurückzukehren. Hierdurch wird eine senkrecht auf das Bindegewebe gerichtete leichte Druckwirkung im Bereich der Falten 35, 36 erreicht, gegebenenfalls auch eine geringe seitliche Druckwirkung. Darüber hinaus wird durch die Thermofolie 5 eine Wärmewirkung erzielt. Die hauteigene Wärme wird hierdurch innerhalb des Pflasters gehalten, wodurch sich wiederum die Poren öffnen und ein gegebenenfalls aufgetragenes kosmetisches Mittel deutlich besser durch die Poren in die Haut eindringen kann.

Durch die leichte Druckwirkung werden Zellen und Gewebeflüssigkeit in den Bereich der Falte gedrückt.

Fig. 1 zeigt ein Pflaster 37 mit zwei Vorsprüngen 38, 39, wobei das Pflaster im Oberlippenbereich 40 eines Gesichtes 41 angeordnet ist. Darüber hinaus sind unterhalb der Augenlider Pflaster 43, 44 angeordnet, die jeweils nur einen Vorsprung 45, 46 aufweisen, wobei der jeweilige Vorsprung etwa parallel zu Falten (nicht gezeigt) unterhalb des Augenlids und im Abstand von zirka 4 bis 8 mm von den Falten verläuft.

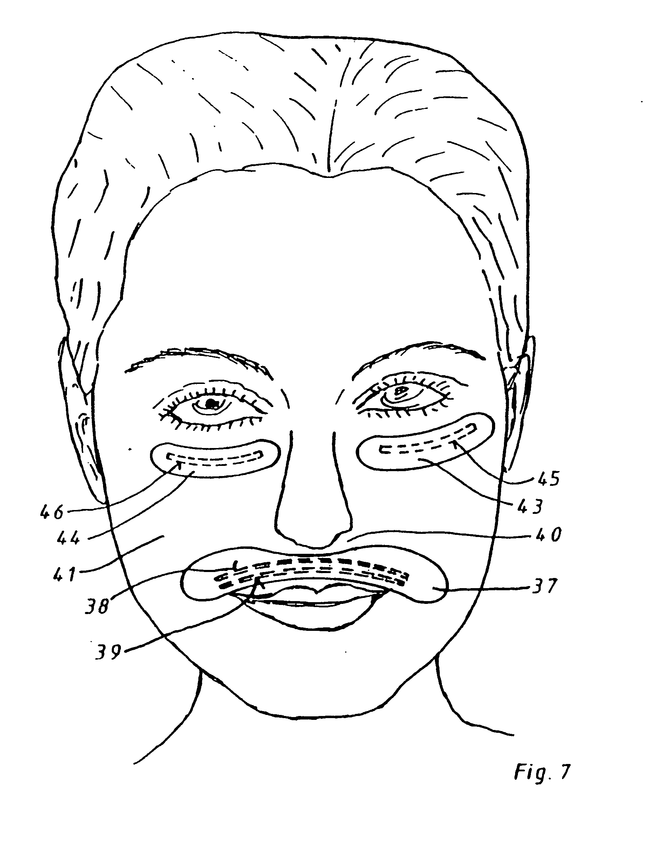

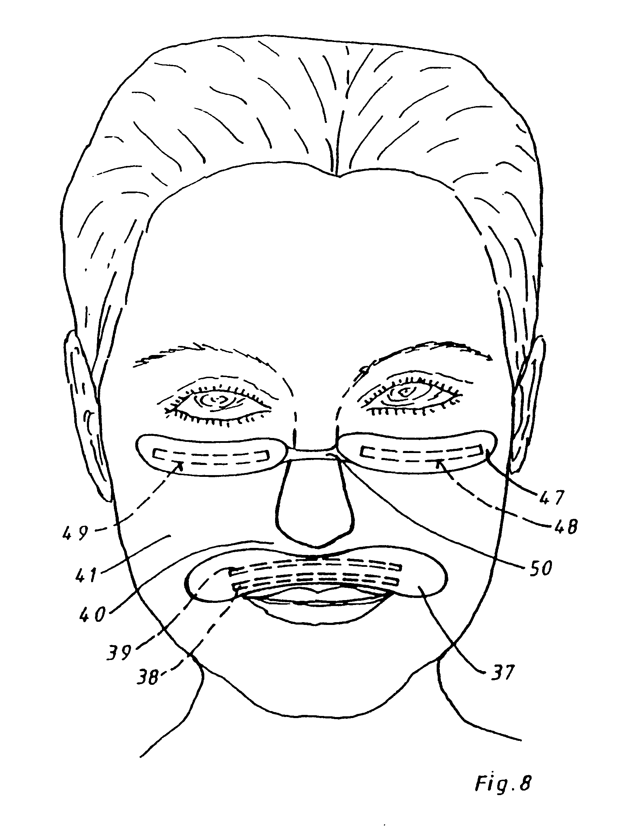

Gemäß Fig. 8 ist ein Pflaster 47 unterhalb der Augen angeordnet. Das Pflaster 47 weist Vorsprünge 48, 49 auf. Das Pflaster 47 weist darüber hinaus einen Nasensteg 50 auf. Die Anordnung der Vorsprünge zu den Falten erfolgt etwa wie gemäß Fig. 7.

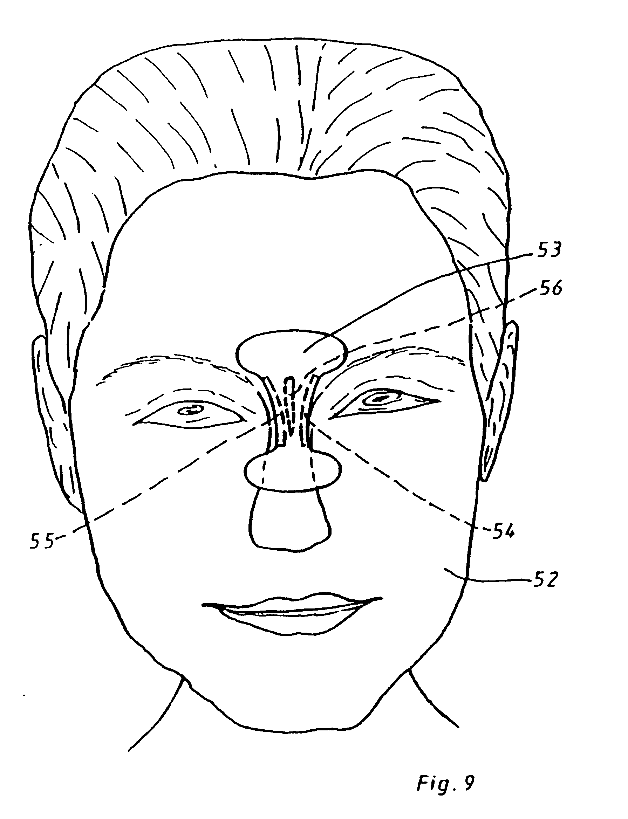

Fig. 9 zeigt ein Gesicht 52 mit einem im oberen Nasenbereich angeordneten Pflaster 53. Das Pflaster 53 weist zwei Vorsprünge in Form von Stegen 54, 55 auf, die beiderseits einer Falte 56 angeordnet sind.

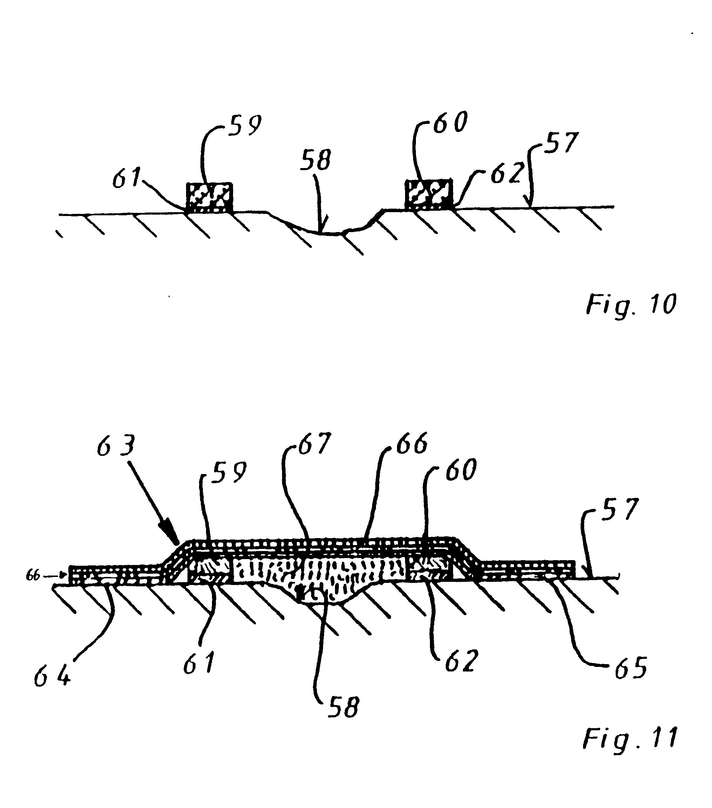

Fig. 10 zeigt eine Hautoberfläche 57 mit einer Falte 58. Rechts und links von der Falte 58 sind von der Trägerfläche des Pflasters lösbare Druckpolster 59, 60 in Form von Streifen angeordnet. Die Streifen 59, 60 weisen jeweils eine selbstklebende Schicht 61, 62 auf, derart, daß die Streifen 59, 60 jeweils links und rechts neben der Falte 58 auf der Haut 57 angeordnet werden können und dort haften bleiben.

Anschließend wird gemäß Fig. 11 die Trägerfläche in Form des Pflasters 63 über die Streifen 59, 60 und die Falte 58 geklebt. Das Pflaster weist hierzu Klebeschichten 64, 65 auf. Durch das Andrücken des Pflasters d.h. der Trägerflächen werden die streifen 59, 60 zusammengedrückt, derart, daß eine Druckwirkung auf die Haut 57 einschließlich des darunter liegenden Bindegewebes und zugleich auf die benachbarte Falte 58 entsteht.

Das Pflaster 63 weist darüber hinaus eine wärmeisolierende Schicht 66 zwischen Trägerschicht und Klebeschicht 64, 65 auf, um die Wärme der Haut im Bereich des Pflasters zu halten. Weiterhin ist zwischen der Schicht 66 bzw. der Klebeschicht 64, 65, den Streifen 59, 60 und der Haut 57 ein kosmetisches Mittel, beispielsweise als Anti-Faltencreme 67 angeordnet.

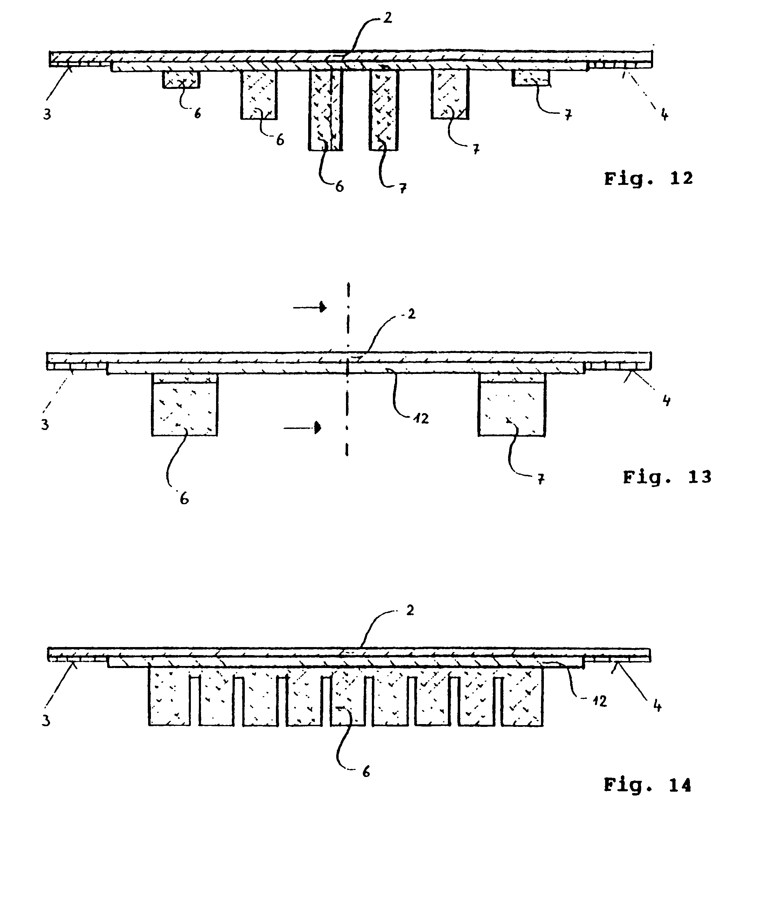

In Fig. 12 ist eine Ausführungsform mit graduell abgestuften Vorsprüngen gezeigt, wobei das Pflaster so angeordnet ist, daß die zu behandelnde Hautfalte zwischen den beiden höchsten Vorsprüngen liegt.

Fig. 13 ist die Vorderansicht im schnitt und Fig. 14 die Seitenansicht im Schnitt vom Pflaster mit Vorsprüngen, die quer zu den beiden länglichen Vorsprüngen Vertiefungen (Rillen) aufweisen, wobei die Rillen unterschiedlich tief sein können und unterschiedliche Abstände voneinander aufweisen können.

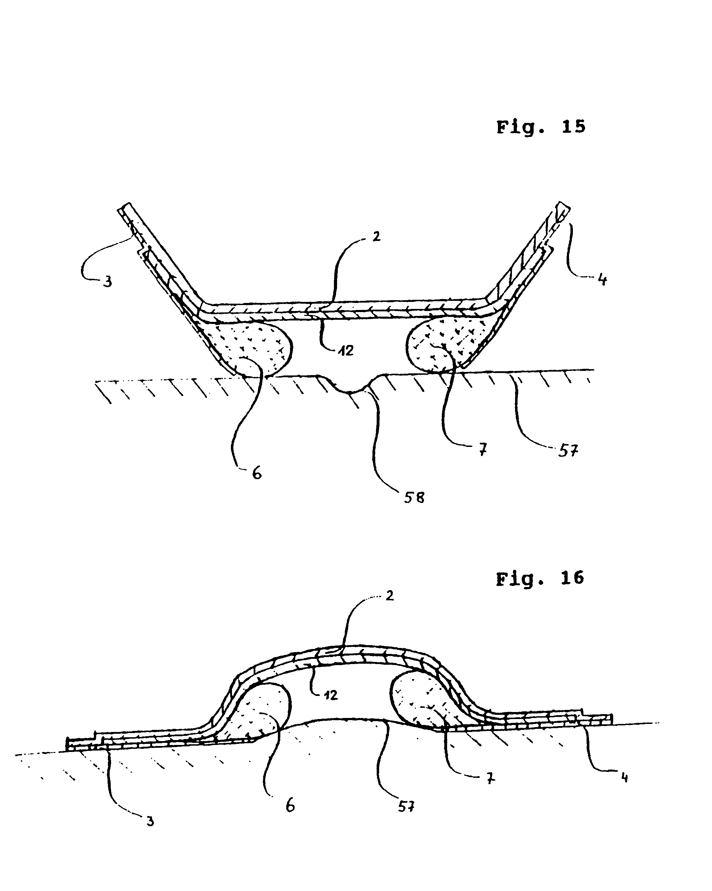

Fig. 15 und 16 sind bereits oben erläutert worden.

### Bezugszahlen

- 1: Pflaster
- 2: Textilschicht
- 3, 4: Klebeschicht
- 5: Metallfolie
- 6, 7: Vorsprung
- 8, 9: Draht
- 10: Pflaster
- 11: Textilschicht
- 12: Thermofolie
- 13, 14: Vorsprünge
- 15: Feld für Creme
- 16: Anti-Faltencreme
- 17, 18: Klebeschichten
- 19: Pflaster
- 20: Vorsprung
- 21, 22: Klebeschichten
- 23: Thermofolie
- 24: Pflaster
- 25: Textilschicht
- 26: Thermofolie
- 27, 28: Klebeschichten
- 29: Vorsprung
- 30: Pflaster
- 31: Thermofolie
- 32, 33: Klebeschichten
- 34: Vorsprung
- 35: Falte
- 36: Falte
- 37: Pflaster
- 38, 39: Vorsprünge
- 40: Oberlippenbereich
- 41, 42: Gesicht
- 43, 44: Pflaster
- 45, 46: Vorsprünge
- 47: Pflaster
- 48, 49: Vorsprünge
- 50: Nasensteg
- 51: Textilschicht
- 52: Gesicht
- 53: Pflaster
- 54: Steg
- 55: Steg
- 56: Falte
- 57: Hautoberfläche
- 58: Falte
- 59, 60: streifen (Druckpolster)
- 61, 62: selbstklebende Schichten
- 63: Pflaster
- 64, 65: Klebeflächen
- 66: wärmeisolierende Schicht
- 67: Anti-Faltencreme

## Patentansprüche

1. Kosmetisches Pflaster zum Aufbringen auf menschliche Haut mit einer Haftschicht oder sekundären Befestigungen und mit Vorsprüngen auf der der Haut zugewandten Innenseite des Pflasters, wobei der oder die Vorsprünge durch einen länglichen Vorsprung (20, 34, 45, 46, 48, 49) oder zwei bis vier dicht beieinander und im wesentlichen parallel zueinander angeordnete längliche Vorsprünge (6, 7, 13, 14, 29, 38, 39, 54, 55, 59, 60) verkörpert werden, die als elastisches, komprimierbares Druckpolster für die Haut und das darunterliegende Bindegewebe ausgebildet sind, und das Pflaster (19) aus einem thermisch isolierenden Material (23) besteht oder eine thermisch isolierende Schicht (5, 12, 26, 31, 66) zwischen der Haftschicht und den anderen Teilen des Pflasters (1, 10, 24, 30, 37, 43, 44, 47, 53, 63) angeordnet ist.

2. Pflaster nach Anspruch 1, wobei die thermisch isolierende Schicht (12, 26, 31, 66) zwischen der Unterseite der Vorsprünge (13, 14, 29, 34, 59, 60) und einer Haftschicht (17, 18, 27, 28, 32, 33, 61 62) einerseits und einer Trägerschicht (11, 25, 51) andererseits angeordnet ist.

3. Pflaster nach Anspruch 1, wobei die thermisch isolierende Schicht zwischen einer Haftschicht einerseits und der Oberseite der Vorsprünge und einer Trägerschicht andererseits angeordnet ist.

4. Pflaster nach Anspruch 1, wobei zwei im wesentlichen parallel zueinander angeordnete längliche Vorsprünge (6, 7, 13, 14, 29, 38, 39, 54, 55) einen Abstand im Bereich von 1-30 mm haben.

5. Pflaster nach Anspruch 1, wobei auf einem Pflaster mehrere einzelne längliche Vorsprünge (45, 46, 48, 49) und/oder mehrere parallel zueinander stehende längliche Vorsprünge (38, 39) entsprechend dem Faltenbild einer menschlichen Gesichtshaut angeordnet sind

6. Pflaster nach einem der Ansprüche 1 bis 5, wobei die Vorsprünge (6, 7, 13, 14, 20, 29, 34, 38, 39, 45, 46, 48, 49, 54, 55, 59, 60) leicht lösbar mit dem Pflaster verbunden sind.

7. Verwendung eines kosmetischen Pflasters nach einem der Ansprüche 1 bis 6 zum Aufkleben auf die Haut in der Weise, daß die wenigstens ein länglicher Vorsprung in Nähe einer Hautfalte und im wesentlichen parallel zu dieser Hautfalte ausgerichtet oder angeordnet wird und das Pflaster mit der Innenseite in dieser Stellung angedrückt und für einen Zeitraum von 30 Minuten bis 24 stunden in dieser Stellung befestigt, vorzugsweise festgeklebt wird, wobei der Vorsprung entweder mit dem Pflaster verbunden ist oder getrennt vom Pflaster vorliegt, und das Pflastermaterial nach der Anordnung des getrennt vorliegenden Vorsprunges neben einer Hautfalte anschließend über dem Vorsprung positioniert und wenigstens auf der Haut befestigt wird.

8. Verwendung eines kosmetischen Pflasters nach einem der Ansprüche 1 bis 7 zum Aufkleben auf die Haut in der Weise, daß zwei im wesentlichen parallel zueinander stehende längliche Vorsprünge links und rechts von einer Hautfalte und im wesentlichen parallel zu dieser Hautfalte angeordnet oder ausgerichtet werden und das Pflaster mit der Innenseite in dieser Stellung angedrückt und für einen Zeitraum von 30 Minuten bis 24 Stunden in dieser Stellung befestigt, insbesondere festgeklebt wird, wobei die Vorsprünge entweder mit dem Pflaster verbunden sind oder getrennt vom Pflaster vorliegen, und das Pflastermaterial nach der Anordnung der getrennt vorliegenden Vorsprünge links und rechts neben einer Hautfalte anschließend über den Vorsprüngen positioniert und wenigstens auf der Haut befestigt wird.

9. Verwendung nach Anspruch 7 oder 8, **dadurch gekennzeichnet, daß** das Andrücken des Pflasters unter gleichzeitiger Kompression des elastischen oder weichelastischen Materials der Vorsprünge erfolgt.

10. Kosmetisches Verfahren zur Rückbildung von Hautfalten, **dadurch gekennzeichnet, daß** man ein Pflaster gemäß Anspruch 1 bis 6 in Nähe der Hautfalte und mit den länglichen Vorsprüngen des Pflasters im wesentlichen parallel zu der Hautfalte aufbringt oder durch die Hände oder ein Massagegerät auf die Stellen neben der Hautfalte Druck ausübt oder beide Maßnahmen nacheinander und mehrfach ausführt.

## Claims

1. A cosmetic plaster for application to human skin with an adhesive layer or secondary fixing elements and with protrusions on the inner surface of the plaster, which faces the skin, wherein the protrusion or protrusions are embodied by one oblong protrusion (20, 34, 45, 46, 48, 49) or two to four oblong protrusions (6, 7, 13, 14, 29, 38, 39, 54, 55, 59, 60) that are closely adjacent and arranged essentially parallel to one another, which protrusion(s) is/are designed as elastic, compressible cushioning for the skin and the underlying connective tissue, and the plaster (19) consists of a thermally insulating material (23), or a thermally insulating layer (5, 12, 26, 31, 66) is arranged between the adhesive layer and the other parts of the plaster (1, 10, 24, 30, 37, 43, 44, 47, 53, 63).

2. A plaster according to claim 1, wherein the thermally insulating layer (12, 26, 31, 66) is arranged between the lower surface of the protrusions (13, 14, 29, 34, 59, 60) and an adhesive layer (17, 18, 27, 28, 32, 33, 61, 62) on the one side, and a support layer (11, 25, 51) on the other side.

3. A plaster according to claim 1, wherein the thermally insulating layer is arranged between an adhesive layer on the one side, and the upper surface of the protrusions and a support layer on the other side.

4. A plaster according to claim 1, wherein two oblong protrusions (6, 7, 13, 14, 29, 38, 39, 54, 55) arranged essentially parallel to one another are at a distance in the range of 1-30 mm from one another.

5. A plaster according to claim 1, wherein multiple separate oblong protrusions (45, 46, 48, 49) and/or multiple oblong protrusions (38, 39) lying parallel to one another are arranged on one plaster so as to correspond to the wrinkle pattern of human facial skin.

6. A plaster according to any one of claims 1 through 5, wherein the protrusions (6, 7, 13, 14, 20, 29, 34, 38, 39, 45, 46, 48, 49, 54, 55, 59, 60) are attached to the plaster so as to be easily detachable.

7. The use of a cosmetic plaster according to any one of claims 1 through 6 for affixing to the skin in such a manner that the at least one oblong protrusion is aligned or arranged near a wrinkle and essentially parallel to said wrinkle and said plaster, with the inner surface in this position, is pressed on and is attached, preferably glued, for a period of time of between 30 minutes and 24 hours, whereby the protrusion is either connected to said plaster, or is separate from said plaster, in which case the plaster material is subsequently positioned over the protrusion after the arrangement of the separate protrusion next to a wrinkle, and is attached at least to the skin.

8. The use of a cosmetic plaster according to any one of claims 1 through 7 for affixing to the skin in such a manner that two oblong protrusions lying essentially parallel to one another are arranged or aligned to the left and right of a wrinkle and essentially parallel to said wrinkle and said plaster, with the inner surface in this position, is pressed on and is attached, especially glued, for a period of time of between 30 minutes and 24 hours, whereby the protrusions are either connected to said plaster, or are separate from said plaster, in which case the plaster material is subsequently positioned over the protrusions after the arrangement of the separate protrusions to the left and right of a wrinkle, and is attached at least to the skin.

9. The use according to claim 7 or 8 **characterized in that** the pressing-on of the plaster takes place under simultaneous compression of the elastic or flexible material of the protrusions.

10. A cosmetic method for the regeneration of wrinkles **characterized in that** a plaster according to claims 1 through 6 is applied near the wrinkle and with the oblong protrusions of said plaster essentially parallel to the wrinkle, or pressure is exerted using the hands or a massage device on the sites adjacent to the wrinkle, or both steps are performed repeatedly and in succession.

## Revendications

1. Pansement cosmétique destiné à être appliqué sur de la peau humaine avec une couche adhésive ou des fixations secondaires et avec des protubérances sur le côté intérieur tourné vers la peau, dans lequel la ou les protubérances sont incarnées par une protubérance de forme allongée (20, 34, 45, 46, 48, 49) ou par deux à quatre protubérances de forme allongée (6, 7, 13, 14, 29, 38, 39, 54, 55, 59, 60) disposées très près les unes des autres et sensiblement parallèles les unes aux autres, qui sont configurées comme un coussin de compression élastique comprimable pour la peau et le tissu conjonctif qui se trouve en dessous et le pansement (19) se compose d'un matériau calorifuge (23) ou bien une couche calorifuge (5, 12, 26, 31, 66) est disposée entre la couche adhésive et les autres parties du pansement (1, 10, 24, 30, 37, 43, 44, 47, 53, 63).

2. Pansement selon la revendication 1, dans lequel la couche calorifuge (12, 26, 31, 66) est disposée entre le côté inférieur des protubérances (13, 14, 29, 34, 59, 60) et une couche adhésive (17, 18, 27, 28, 32, 33, 51, 62) d'une part et une couche de support (11, 25, 51) d'autre part.

3. Pansement selon la revendication 1, dans lequel la couche calorifuge est disposée entre une couche adhésive d'une part et le côté supérieur des protubérances et une couche de support d'autre part.

4. Pansement selon la revendication 1, dans lequel deux protubérances de forme allongée (6, 7, 13, 14, 29, 38, 39, 54, 55) disposées sensiblement parallèles l'une par rapport à l'autre sont espacées de 1 à 30 mm.

5. Pansement selon la revendication 1, dans lequel plusieurs protubérances individuelles de forme allongée (45, 46, 48, 49) et/ou plusieurs protubérances de forme allongée (38, 39) parallèles les unes par rapport aux autres conformément à l'image des rides d'une peau de visage humain sont disposées sur un pansement.

6. Pansement selon l'une des revendications 1 à 5, dans lequel les protubérances (6, 7, 13, 14, 20, 29, 34, 35, 39, 45, 46, 48, 49, 54, 55, 59, 60) sont reliées au pansement de manière facilement amovible.

7. Utilisation d'un pansement cosmétique selon l'une des revendications 1 à 6 destiné à être collé sur la peau de telle manière qu'au moins une protubérance de forme allongée est alignée ou disposée à proximité d'un pli cutané et sensiblement parallèlement à ce pli cutané et que le pansement est comprimé avec le côté intérieur dans cette position et fixé dans cette position pendant un laps de temps de 30 minutes à 24 heures, de préférence collé, sachant que la protubérance est soit reliée au pansement soit se trouve séparée du pansement et que le matériau du pansement est positionné ensuite d'après la disposition de la protubérance séparée à côté d'un pli cutané au-dessus de la protubérance et au moins fixé sur la peau.

8. Utilisation d'un pansement cosmétique selon l'une des revendications 1 à 7 destiné à être collé sur la peau de telle manière que deux protubérances de forme allongée sensiblement parallèle l'une par rapport à l'autre sont disposées ou alignées à droite et à gauche d'un pli cutané et sensiblement parallèlement à ce pli cutané et que le pansement est comprimé avec le côté intérieur dans cette position et fixé dans cette position pendant un laps de temps de 30 minutes à 24 heures, notamment collé, sachant que les protubérances sont soit reliées au pansement soit se trouvent séparées du pansement et que le matériau du pansement est positionné ensuite d'après la disposition des protubérances séparées à droite et à gauche, à côté d'un pli cutané au-dessus des protubérances et au moins fixé sur la peau.

9. Utilisation selon la revendication 7 ou 8, **caractérisé en ce que** la pression du pansement se produit par une compression simultanée du matériau élastique ou souple des protubérances.

10. Procédé cosmétique pour la régression des plis cutanés, **caractérisé en ce que** l'on applique un pansement selon la revendication 1 à 6 à proximité du pli cutané et avec des protubérances de forme allongée du pansement sensiblement parallèlement au pli cutané ou que l'on exerce une pression avec les mains ou avec un appareil de massage sur les points à côté du pli cutané ou que l'on applique les deux mesures l'une après l'autre et plusieurs fois.
